# EUROPEAN PATENT SPECIFICATION

(11) **EP 3 510 153 B1**
(45) Date of publication and mention of the grant of the patent: **07.06.2023**
(21) Application number: 17777180.5
(22) Date of filing: 12.09.2017
(51) Int. Cl.: C12N 15/10, C12Q 1/68

(54) **METHODS AND COMPOSITIONS FOR PURIFYING DOUBLE STRANDED NUCLEIC ACIDS**
METHODEN UND KOMPOSITIONEN ZUR REINIGUNG VON DOPPELSTRÄNGIGEN NUKLEINSÄUREN
MÉTHODES ET COMPOSITIONS POUR PURIFIER DES ACIDES NUCLÉIQUES DOUBLE BRIN

(30) Priority: 12.09.2016 US 201662393331 P
(43) Date of publication of application: 17.07.2019
(73) Proprietor: Roche Diagnostics GmbH, 68305 Mannheim (DE); F. Hoffmann-La Roche AG, 4070 Basel (CH)
(72) Inventor: BELLARE, Priya, Pleasanton, California 94588 (US); HOLCOMB, Cherie, Oakland, California 94605 (US); CASILLA, Alan, Pleasanton, California 94588 (US); HRYCE, Trevor, Pleasanton, California 94588 (US); RUAN, Qiao, Pleasanton, California 94588 (US); KAMPANI, Karan, Pleasanton, California 94588 (US); KOZLOV, Igor, Pleasanton, California 94588 (US)
(74) Representative: Hövelmann, Sascha Alexander
(86) International application number: PCT/EP2017/072831
(87) International publication number: WO 2018/046748

(56) References cited:
- EP-A1- 1 510 577
- EP-A1- 1 748 072
- WO-A1-2014/122288
- WO-A2-2013/003376
- US-A1- 2002 132 244
- US-A1- 2003 199 078
- US-B1- 6 255 477
- DEGENKOLBE T ET AL: "A quality-controlled microarray method for gene expression profiling", ANALYTICAL BIOCHEMISTRY, ELSEVIER, AMSTERDAM, NL, vol. 346, no. 2, 15 November 2005 (2005-11-15), pages 217-224, XP027219305, ISSN: 0003-2697 [retrieved on 2005-10-28]

## Description

### BACKGROUND OF THE INVENTION

Magnetic glass beads are conveniently used for nucleic acid purification. Recently, focus has been on obtaining nucleic acids from samples obtained in a minimally-invasive or non-invasive manner, *e.g*., from blood, saliva, urine, etc. Cell free nucleic acids (cfNAs) in these samples can include relatively short polynucleotides, *e.g*., less than 100 or 1000 bp, that are not efficiently recovered with magnetic glass beads using standard binding and elution conditions. Moreover, insufficient double stranded cfNAs, which are required for some analytical techniques, are obtained using standard conditions.

Such standard methods are, e.g., disclosed in the following documents. WO2013/003376 discloses methods for segregating target nucleic acids from mixed nucleic acid samples. EP 1748072 discloses methods for obtaining nucleic acids using particular hydroxysaline coated MGPs, wherein DNA elution is performed at a temperature of 80°C. US 6,255,477 discloses the use of MGPs having a glass surface for isolating nucleic acids, wherein DNA elution is performed at a temperature of 70°C. However, neither of these documents disclose an automated method, wherein cell-free nucleic acids remain double stranded from sample to elution thereby allowing for high recovery of double stranded nucleic acids.

WO2014/122288 discloses a method for isolating DNA, which is bound to a silicon containing surface, wherein the size of DNA binding to the surface is controlled by the pH of the binding mixture. Degenkolbe et al; Analytical Biochemistry (2005), vol. 346, no.2; pages 217-224 discloses a method of gene expression profiling on microarrays using a sample preparation method that utilizes MGPs for binding mRNA, wherein the mRNA is eluated from the beads for 2 minutes at 95°C and wherein the elution step is performed twice. US 2002/132244 discloses a method for detecting low frequency nuclear mutations comprising isolation of genomic DNA using streptavidin-coated beads, wherein after separation the captured DNA is heated for elution and to subsequently reanneal to form double-strands. However, there is no disclosure in any of these documents on an automated method, wherein cell-free nucleic acids remain double stranded from sample to elution thereby allowing for high recovery of double stranded nucleic acids.

US 2003/199078 discloses protocols for isolation of DNA using MGPs that comprises one elution step that is performed at room temperature or alternatively by using a preheated elution buffer having a temperature of 70 or 80°C. EP 1519577 discloses a method for isolating DNA or RNA using silica magnetic beads in a preferably automated process. After automated isolation of genomic DNA a single elution step is performed at room temperature. However, also neither of these documents discloses isolation of cell-free nucleic acids, wherein at least two elution steps are performed at a temperature between 25°C and 45°C nor is there any indication that the cell-free DNA remains double stranded from sample to elution.

### SUMMARY OF THE INVENTION

The scope of the invention is defined by the appended claims.

Provided herein are methods and compositions for automated recovery of double stranded nucleic acids from magnetic glass beads (MGPs). The methods comprise eluting double stranded nucleic acids non-covalently bound to MGPs from the MGPs using two or more low temperature elutions. Herein, an automated method for recovering double-stranded nucleic acids from a liquid sample is provided, that comprises a) contacting a liquid sample containing double-stranded nucleic acids with magnetic glass particles (MGPs) in chaotropic solution at a temperature between 25°C and 45°C in a vessel, thereby allowing the double-stranded nucleic acids to non-covalently bind the MGPs in solution in the vessel; b) applying a magnetic field to the vessel holding double-stranded nucleic acids non-covalently bound to MGPs in solution (e.g., comprising wash buffer or sample with binding buffer) and removing liquid not bound to the MGPs; c) conducting a first elution step, wherein said first elution step comprises (i) contacting double-stranded nucleic acids non-covalently bound to MGPs with elution buffer having a pH of 7-10 in the vessel at a temperature between 25°C and 45°C for 5-30 minutes; (ii) applying a magnetic field to the vessel; and (iii) collecting liquid not bound to the MGPs, thereby collecting eluate; d) conducting a second elution step, wherein said second elution step comprises (i) contacting double-stranded nucleic acids non-covalently bound to MGPs with elution buffer having a pH of 7-10 in the vessel at a temperature between 25°C and 45°C for 5-30 minutes; (ii) applying a magnetic field to the vessel; and (iii) collecting liquid not bound to the MGPs, thereby collecting eluate; wherein the eluate collected in steps c)(iii) and d)(iii) comprises at least 30% double-stranded nucleic acids recovered from the MGPs of the total nucleic acids in the eluate and wherein the double-stranded nucleic acids is cell-free DNA of 50-500 base pairs in length.

The temperature in steps c)(i) and d)(i) is between 25°C and 45°C, e.g., 25°C, 37°C, 42°C, or 45°C. In some embodiments, the double-stranded nucleic acids are from a cell free sample, e.g., selected from the group consisting of blood, plasma, serum, amniotic fluid, cerebrospinal fluid, and urine.

In some embodiments, the solution in steps a) and b) has a pH lower than 7, *e.g.*, 3-6.5 or 3.5-6. In some embodiments, the elution buffer has a pH of 7.5-9.

In some embodiments, the automated method further comprises between steps a) and b): applying a magnetic field to the vessel and removing liquid not bound to the MGPs, thereby leaving double-stranded nucleic acids non-covalently bound to the MGPs; and adding liquid (*e.g*., wash buffer) to the double-stranded nucleic acids non-covalently bound to the MGPs. In some embodiments, the steps of suspending double-stranded nucleic acids non-covalently bound to the MGPs in wash buffer, applying a magnetic field, and removing unbound liquid are carried out more than once (*e.g*., 2, 3, 4, or 5 times) before elution.

In some embodiments, the elution buffer is contacted with the double-stranded nucleic acids non-covalently bound to MGPs for 5-10, 10-30, 5-25, or 7-25 minutes) in steps c)(i) and d)(i). In some embodiments, more than two elutions are carried out, *e.g*., 3, 4, 5, or 6 elutions. In some embodiments, the two or more elutions have different durations, while in other embodiments, the elutions are of the same duration.

In some embodiments, the elution buffer is mixed with the double-stranded nucleic acids non-covalently bound to MGPs using an automated pipette. In some embodiments, the vessel is a well in a multiwell plate. In some embodiments, the vessel is a tube, *e.g*., with a capacity of 1-5 or 1.5-3 mL. In some embodiments, the vessel is a microwell/ microchamber in a chip.

The nucleic acids recovered in the eluate from steps c)(iii) and d)(iii) comprise at least 30% double-stranded nucleic acids (*e.g*., non-denatured, naturally paired). That is, the double-stranded nucleic acids recovered in the eluate comprise at least 30% of the total nucleic acids in the eluate. In some embodiments, the nucleic acids recovered in the eluate from steps c)(iii) and d)(iii) comprise at least 40%, 50%, 60%, 70%, 80%, 85%, 90%, or more double-stranded nucleic acids.

### DETAILED DESCRIPTION OF THE INVENITON

### I. Introduction

The present disclosure provides reagents that enhance the purification of nucleic acids (NA) of small size (*e.g*., <1 kb, 50-2000 bp, 50-500bp, 100-1000bp) from biological sources (*e.g*., plasma, serum, urine, and other body fluids, as well as from cellular samples or lysates) using magnetic glass particles (MGPs). The presently disclosed techniques and compositions allow for recovery of double stranded cfNA in an amount sufficient for downstream analytical techniques such as amplification and sequencing. The development of such reagents and techniques is necessary because with standard nucleic acid isolation reagents, small nucleic acids are not recovered from MGPs and thus, are not purified in appreciable amounts. The presently disclosed reagents and methods overcome the low recovery issue of circulating NAs from when used in combination with other existing reagents in nucleic acid preparation (NAP) products. These methods are useful not only with Roche NAP products (*e.g*., MagNA Pure reagents), but with other similar systems.

The magnetic glass particles can be used for manual nucleic acid purification (NAP) or for automated NAP on an appropriate specialized system (*e.g*., MagNA Pure 96 or MagNA Pure 24) or general liquid handler (*e.g*., Biomek NX).

Volumes of biological samples used can range from very small (*e.g*., 50 ul) to large (*e.g*., 4 ml or more). Non-limiting examples of uses for the presently described methods and reagents include purification of cell free nucleic acid from plasma or urine as "liquid biopsy" for cancer, non-invasive prenatal testing (NIPT), and early detection of solid organ transplant rejection.

The presently disclosed methods allow for purification of small double stranded nucleic acids using magnetic beads with higher recovery than other automated systems, and even some manual techniques for NAP, *e.g*., spin columns.

### II. Definitions

The term "automated" refers to actions carried out in a programmed manner without direct human action. The automated action can be controlled remotely, programmed, or observed by a technician, but without requiring the technician to manually interfere, *e.g*., use a manual pipette, move vessels by hand, etc.

The term "magnetic glass particle" or "MGP" refers to a particle comprising glass that non-covalently binds nucleic acids, and at least one magnetic core (*e.g*., a dispersion of magnetic cores) that respond to a magnetic field. The glass is not necessarily pure silica, though silica can be a component. MGPs are small enough to be pipetted in a standard pipette tip and form a suspension, typically 0.5-15 um. MGPs are roughly spherical on average, and can be porous or non-porous. The magnetic core can be ferromagnetic or paramagnetic (only magnetized in the presence of a magnetic field). Suitable MGPs are described in more detail, *e.g*., in US Patent Nos. 6,255,477 and 6,545,143.

The terms "solid (or semisolid) matrix," "solid (or semisolid) support" is used herein to denote an inert surface or body to which an agent, such as nucleic acid can be immobilized. Non-limiting examples include glass, silica, plastic, nitrocellulose, membranes, chips, and particles. The term "immobilized" as used herein denotes a molecular-based coupling that is not significantly de-coupled under the conditions imposed during the steps of the assays described herein. Such immobilization can be achieved through a covalent bond, an ionic bond, an affinity-type bond, or any other chemical bond.

The term "in solution," *e.g*., referring to a solid support in solution, can indicate that the solid support is exposed to a solution (*e.g*., in contact with assay components in solution) or that the solid supports themselves are in solution (*e.g*., beads or particles suspended in liquid). The term is used to distinguish between hydrophilic liquid phase, and an emulsion with separate hydrophilic and hydrophobic components.

The terms "receptacle," "vessel," "tube," "well," "chamber," "microchamber," etc. refer to a container that can hold reagents or an assay. If the receptacle is in a kit and holds reagents, or is being used for an amplification reaction, it can be closed or sealed to avoid contamination or evaporation. If the receptacle is being used for an assay, it can be open or accessible, at least during set up of the assay.

The term "capture," "capturing," "bind," or "binding," in the context of an MGP binding nucleic acid, refers to non-covalent binding, e.g., through chaotropic or ionic interaction. The MGP-nucleic acid interaction can be disrupted by elution, *e.g*., using an elution buffer that interferes with the non-covalent interaction.

The terms "nucleic acid," "polynucleotide," and "oligonucleotide" refer to polymers of nucleotides (*e.g*., ribonucleotides or deoxyribo-nucleotides) and includes naturally-occurring (adenosine, guanidine, cytosine, uracil and thymidine), non-naturally occurring, and modified nucleic acids. The term is not limited by length (*e.g*., number of monomers) of the polymer. A nucleic acid may be single-stranded or double-stranded and will generally contain 5'-3' phosphodiester bonds, although in some cases, nucleotide analogs may have other linkages. Monomers are typically referred to as nucleotides. The term "modified nucleotide" refers to a non-naturally occurring nucleotide that contains a modified nitrogenous base, sugar or phosphate group, or that incorporates a non-natural moiety in its structure. Examples of non-natural nucleotides include dideoxynucleotides, biotinylated, aminated, deaminated, alkylated, benzylated and fluorophor-labeled nucleotides.

Double stranded nucleic acids, in the context of the present disclosure, typically refer to non-denatured, naturally paired stretches of DNA that have not been separated and rehybridized (re-matched by Watson Crick base pairing). In liquid biopsy samples, such as blood, plasma, serum, urine, mucous, cerebrospinal fluid, and amniotic fluid, cell free nucleic acids include such double-stranded nucleic acids in lengths ranging from about 40-1000 base pairs in length. Cell free nucleic acids are released from cells, *e.g*., from cells killed by apoptosis, or in extracellular vesicles or exosomes. Double-stranded fragments, as they are released into cell free nucleic acids are usually almost completely matched, *e.g*., with no, or only a few overhanging, non-base paired nucleotides on either end. Double-stranded DNA fragments can also be synthetically produced, *e.g*., by sonication or shearing of genomic DNA. These synthetically produced dsDNA fragments also have largely blunt ends. Double stranded DNA fragments with blunt ends can also be produced by restriction enzyme digestion or amplification. When a mixture of such double stranded fragments are exposed to high temperature, they can become unpaired (melted, denatured, unhybridized), and in reduced temperature, either remain single stranded or rehybridize (re-base pair, renature) with a strand that has a different stretch of sequence or length than the strand it was paired with when released from a cell. The latter can lead to pairing of overlapping fragments, so that there is a longer, mismatched, single stranded portion on either end of the double-stranded section, *i.e*., more overhang. Mismatches with overhangs or single stranded nucleic acids could lead to a reduced yield during downstream processes either by failure to fill in blunt ends before ligation of adaptor sequence (*e.g*., for sequencing) or in purification methods with more efficient recovery of dsDNA than single stranded. Alternatively, mispairing can occur at a limited number of internal sites, e.g., where one strand has a mutation and the other does not. Such single base pair mismatches are incompatible with downstream next generation sequencing, in which strand agreement is checked to assure accuracy.

The term "primer" refers to a short single stranded nucleic acid (an oligonucleotide) that acts as a point of initiation of polynucleotide strand synthesis by a nucleic acid polymerase under suitable conditions. Polynucleotide synthesis and amplification reactions typically include an appropriate buffer, dNTPs and/or rNTPs, and one or more optional cofactors, and are carried out at a suitable temperature. A primer typically includes at least one target-hybridized region that is at least substantially complementary to the target sequence (*e.g*., having 0, 1, 2, or 3 mismatches). This region of is typically about 8 to about 40 nucleotides in length, *e.g*., 12-25 nucleotides. As used herein, "probe" means any molecule that is capable of selectively binding to a specifically intended target biomolecule, for example, a nucleic acid sequence of interest to be bound, captured or hybridized by the probes.

The words "complementary" or "complementarity" refer to the ability of a nucleic acid in a polynucleotide to form a base pair with another nucleic acid in a second polynucleotide. For example, the sequence A-G-T (A-G-U for RNA) is complementary to the sequence T-C-A (U-C-A for RNA). Complementarity may be partial, in which only some of the nucleic acids match according to base pairing, or complete, where all the nucleic acids match according to base pairing. A probe or primer is considered "specific for" a target sequence if it is at least partially complementary to the target sequence. Depending on the conditions, the degree of complementarity to the target sequence is typically higher for a shorter nucleic acid such as a primer (*e.g*., greater than 80%, 90%, 95%, or higher) than for a longer sequence.

The terms "identical" or "percent identity," in the context of two or more nucleic acids, or two or more polypeptides, refer to two or more sequences or subsequences that are the same or have a specified percentage of nucleotides, or amino acids, that are the same (*e.g*., about 60% identity, e.g., at least any of 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection. See e.g., the NCBI web site at ncbi.nlm.nih.gov/BLAST. Such sequences are then said to be "substantially identical." Percent identity is typically determined over optimally aligned sequences, so that the definition applies to sequences that have deletions and/or additions, as well as those that have substitutions. The algorithms commonly used in the art account for gaps and the like. Typically, identity exists over a region comprising an a sequence that is at least about 8-25 amino acids or nucleotides in length, or over a region that is 50-100 amino acids or nucleotides in length, or over the entire length of the reference sequence.

The term "kit" refers to any manufacture (*e.g*., a package or a container) including at least one reagent, such as a nucleic acid probe or probe pool or the like, for specifically amplifying, capturing, tagging/converting or detecting RNA or DNA as described herein.

The term "amplification conditions" refers to conditions in a nucleic acid amplification reaction (*e.g*., PCR amplification) that allow for hybridization and template-dependent extension of the primers. The term "amplicon" or "amplification product" refers to a nucleic acid molecule that contains all or a fragment of the target nucleic acid sequence and that is formed as the product of in vitro amplification by any suitable amplification method. One of skill will understand that a forward and reverse primer (primer pair) defines the borders of an amplification product. The term "generate an amplification product" when applied to primers, indicates that the primers, under appropriate conditions (*e.g*., in the presence of a nucleotide polymerase and NTPs), will produce the defined amplification product. Various PCR conditions are described in PCR Strategies (Innis et al., 1995, Academic Press, San Diego, CA) at Chapter 14; PCR Protocols : A Guide to Methods and Applications (Innis et al., Academic Press, NY, 1990).

The term "amplification product" refers to the product of an amplification reaction. The amplification product includes the primers used to initiate each round of polynucleotide synthesis. An "amplicon" is the sequence targeted for amplification, and the term can also be used to refer to amplification product. The 5' and 3' borders of the amplicon are defined by the forward and reverse primers.

The term "sample" or "biological sample" refers to any composition containing or presumed to contain nucleic acid. The term includes purified or separated components of cells, tissues, or blood, *e.g*., DNA, RNA, proteins, cell-free portions, or cell lysates. In the context of the present disclosure, the sample is liquid, *e.g*., blood or a blood component (plasma or serum), urine, semen, saliva, sputum, mucus, semen, tear, lymph, cerebral spinal fluid, mouth/throat rinse, bronchial alveolar lavage, material washed from a swab, etc. Samples also may include constituents and components of *in vitro* cultures of cells obtained from an individual, including cell lines. The liquid sample can also be partially processed from a sample directly obtained from an individual, *e.g*., cell lysate or blood depleted of red blood cells.

In the context of the present disclosure, the term "unbound liquid" or "unbound sample" refers to liquid and other components (*e.g*., proteinaceous material or cell debris) that is not bound to the solid support or MGP, *e.g*., liquid depleted of nucleic acids or other target. The unbound liquid may still include a residual amount of nucleic acids or target.

Extracellular vesicles, including exosomes, microvesicles, and apoptotic bodies, are cell-derived vesicles (membrane-enclosed body) present in biological fluids, *e.g*., blood and urine. Extracellular vesicles can be released from cells, *e.g*., directly from the plasma membrane, or formed when multivesicular bodies fuse with the plasma membrane. Extracellular vesicles typically include components such as nucleic acids and proteins from their cell of origin. Exosomes are typically 40-120 nm in diameter, microvesicles are typically 50-1000 nm in diameter, and apoptotic bodies are typically 500-2000 nm in diameter.

A "control" sample or value refers to a value that serves as a reference, usually a known reference, for comparison to a test sample or test conditions. For example, a test sample can be taken from a test condition, *e.g*., from an individual suspected of having cancer, and compared to samples from known conditions, *e.g*., from a cancer-free individual (negative control), or from an individual known to have cancer (positive control). A control can also represent an average value or a range gathered from a number of tests or results. A control can also be prepared for reaction conditions. For example, a positive control for the presence of nucleic acid could include primers or probes that will detect a sequence known to be present in the sample (*e.g*., a housekeeping gene such as beta actin, beta globin, DHFR, or succinate dehydrogenase, or a known added polynucleotide, *e.g*., having a designated length). An example of a negative control is one free of nucleic acids, or one including primers or probes specific for a sequence that would not be present in the sample, *e.g*., from a different species. One of skill will understand that the selection of positive and negative control will depend on the particular assay, *e.g*., so that the control is cell type and organism-appropriate. One of skill in the art will recognize that controls can be designed for assessment of any number of parameters. For example, a control can be devised to compare therapeutic benefit based on pharmacological data (*e.g*., half-life) or therapeutic measures (*e.g*., comparison of benefit and/or side effects). Controls can be designed for in vitro applications, *e.g*., a known sequence in a known amount. One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

The terms "label," "tag," "detectable moiety," and like terms refer to a composition detectable by spectroscopic, photochemical, biochemical, immunochemical, chemical, or other physical means. For example, useful labels include fluorescent dyes, luminescent agents, radioisotopes (e.g., ³²P, ³H), electron-dense reagents, or an affinity-based moiety, *e.g*., a poly-A (interacts with poly-T) or poly-T tag (interacts with poly-A), a His tag (interacts with Ni), or a strepavidin tag (separable with biotin).

Unless defined otherwise, technical and scientific terms used herein have the same meaning as commonly understood by a person of ordinary skill in the art. See, *e.g*., Lackie, DICTIONARY OF CELL AND MOLECULAR BIOLOGY, Elsevier (4th ed. 2007); Sambrook et al., MOLECULAR CLONING, A LABORATORY MANUAL, Cold Springs Harbor Press (Cold Springs Harbor, N.Y. 1989). The term "a" or "an" is intended to mean "one or more." The terms "comprise," "comprises," and "comprising," when preceding the recitation of a step or an element, are intended to mean that the addition of further steps or elements is optional and not excluded.

### III. Nucleic acid samples

Samples for nucleic acid amplification can be obtained from any source suspected of containing nucleic acid. In some embodiments, the sample is obtained in a non-invasive manner, *e.g*., from urine, skin, swab, saliva, blood or a blood fraction. Samples can also be taken from a tissue biopsy, formalin fixed paraffin embedded tissue (FFPET), or cultured cells. Methods for isolating nucleic acids from biological samples are known, *e.g*., as described in Sambrook, and several kits are commercially available (*e.g.,* DNA Isolation Kit for Cells and Tissues, DNA Isolation Kit for Mammalian Blood, High Pure FFPET DNA Isolation Kit, High Pure RNA Isolation Kit, High Pure Viral Nucleic Acid Kit, and MagNA Pure LC Total Nucleic Acid Isolation Kit, available from Roche).

The present methods can also be applied to liquids, *e.g*., prepared from food or plant samples or from wipes of surfaces, *e.g*., in a hospital setting.

In a sample that includes cells, the cells can be separated out (*e.g*., using size-based filtration or centrifugation), thereby leaving cell free nucleic acids (cfNA), including nucleic acids in exosomes, microvesicles, viral particles, or those circulating freely. Alternatively, the cells can be lysed to obtain cellular nucleic acids, either in the presence of MGPs or before addition of the cellular lysate to the MGPs.

Lysis buffer (LB) that can be used for nucleic acid isolation can include 3-6 M GuSCN or urea, 0-500 mM Buffer (Tris-HCl, MOPS, HEPES, etc.), 10-25% detergent (e.g., Tween, Triton-X, or polydocanol, etc.), 0-200 mM sodium citrate, at pH 5.0-8. While LB refers to lysis, it can be used in cell-free samples such as plasma or serum, and can act to lyse extracellular vesicles, exosomes or microvesicles, if present.

### IV. Solid supports and magnetic glass particles (MGPs)

The presently described nucleic acid preparation methods involve use of a solid or semisolid support, *e.g.,* particle (*e.g.*, microparticles or beads). The size range of the microparticles can vary and particular size ranges are not critical. In most cases, the aggregated size range of the microparticles lies within the range of from about 5 nanometers to about 500 micrometers in particle diameter, *e.g*., within the range of from about 500 nanometers to about 50 micrometers, about 1micrometer to about 100 micrometers, or about 1 micrometer to 30 micrometers.

Magnetic glass particles (MGPs) are known in the art, and are described, *e.g*., in US Patent Nos. 6,255,477 and 6,545,143. The particles used for the presently described methods are typically ferromagnetic. The particles are on average roughly spherical with a diameter of 0.5-15 um (*e.g*., 1-10, 0.8-2, or 1-1.5 um). In some embodiments, the particles are non-porous.

The MGPs can have a single magnetic core coated by glass, or comprise glass infused with several magnetic objects. The magnetic substance can be iron or iron oxide as magnetite (Fe3O4) or Fe2O3 (*e.g*., gamma-Fe2O3). Barium ferrite, nickel, cobalt, Al-Ni-Fe-Co alloys or other ferromagnetic substances can be used. Metal oxides can also be included in the magnetic core, *e.g*., aluminum oxide, iron oxide, chromium oxide, copper oxide, manganese oxide, lead oxide, tin oxide, titanium oxide, zinc oxide, or zirconium oxide.

The glass component is typically silica based, *e.g*., silicon oxide and glass powder, alkylsilica, aluminum silicate, or, NH2-activated silica. In some embodiments, the glass comprises at least one metal oxide (*e.g*., SiO₂, B₂O₃, Al₂O₃, K₂O, CaO, and/or ZnO). In some embodiments, the glass comprises SiO₂, B₂O₃, Al₂O₃, K₂O, and CaO, in order of molar percentage. In some embodiments, the glass comprises SiO₂, B₂O₃, Al₂O₃, K₂O, CaO, and ZnO, in order of molar percentage. In some embodiments, in addition to SiO₂, the glass can include B₂O₃ (0-30%), Al₂O₃ (0-20%), CaO (0-20%), BaO (0-10%), K₂O (0-20%), NazO (0-20%), MgO (0-18%), Pb₂O₃ (0-15%), ZnO (0-6%). In some embodiments, the glass comprises about 70-75% SiO2, about 14-16% B2O3, about 4-6% Al2O3, about 4-5% K2O, about 2-3% CaO, and about 0-5% ZnO. Appropriate MGPs for the presently disclosed methods are commercially available, *e.g*., in MagNA Pure and **cobas^{®}** 4800 Sample Preparation kits from Roche.

Nucleic acid binds to MGPs in chaotropic solution. Chaotropic solutions can include guanidinium thiocyanate (GuSCN), guanindine hydrochloride, urea, sodium iodite, sodium perchlorate, thiocyanate ion, iodine ion, perchlorate ion, nitrate ion, bromine ion, acetate ion, chlorine ion, fluorine ion, or sulfur ion, or combinations thereof. In some embodiments, the chaotrope is in solution at about 1-10 M, *e.g*., 2-8 or 4-6 M, to allow nucleic acid binding.

Methods of, and instrumentation for, applying and removing a magnetic field as part of an assay are known to those skilled in the art and reported in the literature. Examples include US Patent Nos. 4,141,687 and 4,115,534 and Vlieger et al., Analytical Biochemistry 205:1-7 (1992). Any instrument capable of carrying out magnetic separation using MGPs can be used. Depending on the device, samples can be processed in multivessel cartridges or plates, or in individual vessels. Vessels (*e.g*., processing tubes or wells) for use in automated instruments typically hold a liquid volume of 50 uL to 4 mL, more typically 1-2 mL.

Examples of instruments that can be used for automating the presently disclosed methods include but are not limited to the MagNA Pure instruments (Roche), Dynamag^{®} instruments (Thermo Fisher) QIAsymphony^{®} systems (Qiagen), Biomek^{®} systems (Beckman), and Maxwell^{®} instruments (Promega).

### V. Downstream processing and detection

Nucleic acids are typically eluted from the beads before analysis, though MGPs are compatible with some assays (*e.g*., detection of a labeled probe hybridized to nucleic acid on the MGP, PCR, or where elution occurs as part of the assay, such as Southern blotting). Elution conditions interfere with the non-covalent (*e.g*., chaotropic or ionic) interaction of nucleic acid with the MGP, *e.g*., water, buffer with lower chaotrope concentration than used for binding nucleic acids to the MGPs, and/or elevated temperature, as will be appreciated by one of skill in the art. The term "eluate" refers to a desired analyte (*e.g*., nucleic acids) released in solution from a solid support or other separation matrix.

The purified nucleic acid sample can be used for detection, *e.g*., using next generation sequencing, microarray (RNA or DNA), Southern or Northern Blot, or nucleic acid amplification, *e.g*., using any primer-dependent method. DNA-based methods can be used for amplification and detection, *e.g*., PCR. In some embodiments, real time or quantitative PCR is used (RTPCR or qPCR). qPCR allows for reliable detection and measurement of products generated during each cycle of PCR process. Such techniques are well known in the art, and kits and reagents are commercially available, *e.g*., from Roche Molecular Systems, Life Technologies, Bio-Rad, etc. *See*, *e.g.*, Pfaffl (2010) Methods: The ongoing evolution of qPCR, vol. 50. In some embodiments, the probe portion of the branched primer-probe is dual labeled (*e.g*., a TaqMan, CPT, LNA, or MGB probe) with a quencher and a fluorophore (*see, e.g*., Gasparic et al. (2010) Anal. Bioanal. Chem. 396:2023).

In some embodiments, a preliminary reverse transcription step is carried out (also referred to as RT-PCR, not to be confused with real time PCR). *See*, *e.g*., Hierro *et al.* (2006) 72:7148. The term "qRT-PCR" as used herein refers to reverse transcription followed by quantitative PCR. Both reactions can be carried out in a single tube without interruption, *e.g*., to add reagents. For example, a polyT primer can be used to reverse transcribe all mRNAs in a sample with a polyA tail, or a primer can be designed that is specific for a particular target transcript that will be reverse transcribed into cDNA. Additional RNA-based methods of amplification can also be used, *e.g*., nucleic acid sequence based amplification (NASBA) or transcription mediated amplification (TMA).

Detection devices are known in the art and can be selected as appropriate for the selected labels. Detection devices appropriate for quantitative PCR include the cobas* and Light Cycler^{®} systems (Roche), PRISM 7000 and 7300 real-time PCR systems (Applied Biosystems), etc.

Results can be expressed in terms of a threshold cycle (abbreviated as Ct, Cq, or Cp). A lower Ct value reflects the rapid achievement of a predetermined threshold level, *e.g*., because of higher target nucleic acid concentration or a more efficient amplification. A higher Ct value may reflect lower target nucleic acid concentration, or inefficient or inhibited amplification. The threshold cycle is generally selected to be in the linear range of amplification for a given target. In some embodiments, the Ct is set as the cycle at which the growth signal exceeds a pre-defined threshold line, *e.g*., in relation to the baseline (Ct), or by determining the maximum of the second derivation of the growth curve (Cp). Because Ct and Cp values are typically quite close, they are often used interchangeably. Determination of Ct/ Cq/ Cp is known in the art, and described, *e.g*., in Bustin et al. (2009) Clin. Chem. 55:4 (describing MIQE Guidelines) and US Patent No. 7,363,168.

Additional techniques include quantification, *e.g*., using Qubit (ThermoFisher) or Agilent Bioanalyzer, and sequencing, such as next generation sequencing (NGS) techniques. Certain NGS techniques rely on ligation of blunt ended adaptors to double-stranded DNA fragments. These adaptors can be ligated in turn to adaptors with known sequences to allow for sequence specific priming and amplification/ sequencing (*see*, *e.g*., Poptsova et al. (2014) Nature). Examples include Illumina Genomic DNA Sample Prep, Roche 454 Library Preparation, and Life SOLiD Library Preparation. To maximize the percentage of dsDNA fragments in a sample with 5' phosphate blunt ends, a repair kit can be used, *e.g*., MCLAB Fragmented DNA End Repair Kit.

### VI. Kits

In some embodiments, reagents and materials for carrying out the presently disclosed methods are included in a kit. In some embodiments, the kit includes MGPs that bind nucleic acids, one or more binding buffers, one or more wash buffers, and elution buffer. In some embodiments, the binding buffer includes chaotrope, detergent, and a pH buffering agent (*e.g*., Tris, MOPS, HEPES, PIPES, or a buffering system involving citrate, borate, acetate, etc.). Optionally the binding buffer includes at least one salt, isopropanol and/or proteinase K. The binding buffer can be adjusted depending on the concentration and size of nucleic acids in the sample.

Similarly, wash buffers typically have chaotrope, detergent, and a pH buffering agent, and can be varied depending on the expected amount of non-desired material in the sample. If more than one wash buffer is used, later wash buffers can have lower concentrations of such components so that elution will be more effective and the eluate will not have high concentration of chaotrope, detergent, salt, etc., that could interfere with downstream analysis.

Elution buffers typically have minimal ingredients so that eluate can be used directly for analysis (*e.g*., amplification or sequencing). For example, the elution buffer can include pH buffering agent and/or at least one salt.

In some embodiments, the kit further includes at least one control, *e.g*., nucleic acid fragments of known size and concentration. In some embodiments, the kit further includes consumables, *e.g*., plates or tubes for nucleic acid preparation, tubes for sample collection, etc. In some embodiments, the kit further includes instructions for use, reference to a website, or software.

### VII. Examples

Higher cfNA levels have been observed in the plasma of cancer patients compared to plasma from normal, non-cancer individuals. In addition, short polynucleotides, in the range of about 50-1000 bp, are generally much more prevalent in plasma from cancer patients compared to normal plasma. These cfNAs are useful for diagnostic techniques, *e.g*., to detect disease-related mutations, genetic variants, or gene expression levels, as well as for prenatal genetic testing.

Magnetic glass beads (MGPs) provide a convenient method for purifying nucleic acids in an automated fashion. An automated workflow was devised and applied for the recovery of small DNA fragments, in particular double stranded cfNAs, using Roche MGPs. The presently disclosed methods and composition can be used with similar nucleic acid preparation reagents, *e.g*., lysis or binding buffers that provide chaotropic conditions and MGPs utilizing Boom technology (*see, e.g*., US Patent No. 6,562,569 and Boom (1990) J. Clin. Microbiol. 28:495).

### Example 1

Maximal amounts of nucleic acids bound to MGPs were recovered without disrupting hybridization of double-stranded nucleic acids. Current elution methods are carried out at elevated temperature, *e.g*., 90-100°C, to ensure complete disassociation of nucleic acids from MGPs. Double stranded nucleic acids denature, or become single stranded, at such elevated temperatures. This is especially true of shorter nucleic acids such as cfNAs, which may have a melting temperature as low as about 60°C. As double stranded nucleic acids are required for certain downstream analytical techniques, the hybridization of the double stranded nucleic acids were retained by reducing elution temperature.

Plasma spiked with dsDNAs of known concentration, size, and sequence was used for analysis. The known sequences were GUSB (glucuronidase beta; 86 bp target sequence) and PBGD (porphobilinogen deaminase; 150 bp target sequence). The plasma used was pooled from pregnant women, and thus contained cfNAs from the women, as well as some cfNAs from their fetuses. Additionally, endogenous, cfNA-specific target sequences were selected for analysis: a LINE sequence (present at varying copy numbers in genomic DNA, and serves as a marker for DNA in cfNA), and a 149 bp DYZ (fetal derived Y chromosome sequence that, when present in female plasma, indicates male fetal DNA in her cfNA). For the endogenous targets, precise concentration was not known. For LINE, the calculated yield can be used to compare relative yields from the same starting material (*e.g*., plasma), but is not an absolute quantification.

The procedures were carried out on a MagNA Pure 96 instrument, and a similar but smaller instrument under development, with variations that led to comparable results. For example, plasma was added at about 1:1 (ranging 5:1-1:5) with binding buffer (chaotrope, detergent, proteinase K, and isopropanol) to MGPs. Binding was carried out for 4-8 minutes at 25°C, 37°C, and 45°C. In some cases, multiple aliquots of spiked plasma were added to the MGPs to increase the amount of cfNAs captured. The MGPs were separated from unbound material using a magnetic field, followed by resuspension by pipetting in wash buffer. Several (2-5) washes lacking proteinase K, and with decreasing amounts of detergent and salts were carried out.

Once the MGPs were washed, dsDNA was eluted by using one elution or two, both carried out at low temperature. The one or more elutions were carried out below 50°C (25°C-45°C), for 5-30 minutes each. Each target was amplified and detected using qPCR on the LightCycler^{®} 480 using primers and probes to specifically amplify and detect each target. Percent recovery or yield was calculated using Cp values with comparison to known standards. For the known targets, the control was an amplification carried out with the total amount of that target spiked into the plasma sample, so can be expressed in terms of percentage recovery. For the endogenous target, the amount [ng] recovered could be extrapolated based on amplification of a known amount of a control target sequence. Representative results are shown in Table 1 below.

**Table 1**

| **Spiked Target** | **Single elution yield** [%] | **Double elution yield** [%] | **Double elution yield** [%] |
|---|---|---|---|
| GUSB | 52.9 | 71.2 | 69.2 |
| PBGD | 40.1 | 54.7 | N/D |

| **Endogenous Target** | **Single elution yield [ng]** | **Double elution yield [ng]** | **Double elution yield [ng]** |
|---|---|---|---|
| End | 6.30 | 11.79 | 9.68 |
| DYZ | 0.26 | 0.36 | 0.33 |

The results show that double elution effectively yielded significantly higher amounts of cfNA. Because the elutions were carried out at low temperature, the paired structure of the dsDNA in the sample was maintained, making it available for downstream analysis.

### Example 2

The yield of double-stranded DNA from a single, high temperature elution was determined and compared to a double, low temperature elution. Methods were essentially the same as in Example 1. Pooled plasma was spiked with a known quantity of a double stranded DNA of 150 bp containing a G6PD target sequence. Nucleic acid was purified from the plasma on the Roche MagNA Pure 96, using either a single, high temperature elution (ranging from 90 to 96°C), or a double, low temperature elution (ranging from 30 to 47°C). Resulting eluates were evaluated for yield/ recovery using the ThermoFisher Quant-iT PicoGreen ds DNA assay Kit (to detect double stranded DNA), LINE qPCR (total endogenous single or double stranded DNA), or G6PD qPCR (total spiked single or double stranded DNA). Results are shown in Table 2.

**Table 2**

| **Quantification method (units)** | **High temp. single elution** | **Low temp. double elution** |
|---|---|---|
| qPCR LINE [ng] | 10.8 | 11.3 |
| qPCR G6PD [% recovery] | 87.5 | 65.5 |
| PicoGreen ds DNA assay [ng] | 4.1 | 31.2 |

The data show that while overall recovery of DNA is the same regardless of the elution conditions used, the recovery of DNA in the double stranded state is greatly reduced in high temperature conditions. Again, the LINE results can be used to compare between conditions using the same sample pool, but does not reflect absolute DNA yield. The recovery of the G6PD 150 bp fragment is consistent with the data shown in Table 1 for the double elution, though somewhat reduced from the single, high temperature elution. The PicoGreen ds DNA assay results, however, show that yield of double stranded DNA is greatly increased in the reduced temperature double elution.

## Claims

1. An automated method for recovering double-stranded nucleic acids from a liquid sample, comprising:
a) contacting the liquid sample containing double-stranded nucleic acids with magnetic glass particles (MGPs) in chaotropic solution at a temperature between 25°C and 45°C in a vessel, thereby allowing the double-stranded nucleic acids to non-covalently bind the MGPs in solution in the vessel;
b) applying a magnetic field to the vessel holding double-stranded nucleic acids non-covalently bound to MGPs in solution and removing liquid not bound to the MGPs;
c) conducting a first elution step, wherein said first elution step comprises
(i) contacting double-stranded nucleic acids non-covalently bound to MGPs with elution buffer having a pH of 7-10 in the vessel at a temperature between 25°C and 45°C for 5-30 minutes;
(ii) applying a magnetic field to the vessel; and
(iii) collecting liquid not bound to the MGPs, thereby collecting eluate;
d) conducting a second elution step, wherein said second elution step comprises
(i) contacting double-stranded nucleic acids non-covalently bound to MGPs with elution buffer having a pH of 7-10 in the vessel at a temperature between 25°C and 45°C for 5-30 minutes;
(ii) applying a magnetic field to the vessel; and
(iii) collecting liquid not bound to the MGPs, thereby collecting eluate;
wherein the eluate collected in steps c)(iii) and d)(iii) comprises at least 30% double-stranded nucleic acids recovered from the MGPs of the total nucleic acids in the eluate and wherein the double-stranded nucleic acids is cell-free nucleic acid of 50-500 base pairs in length.

2. The automated method of claim 1, wherein the temperature in steps c)(i) and d)(i) is between 30°C and 45°C.

3. The automated method of any one of claims 1 to 2, wherein the solution in steps a) and b) has a pH less than 7.

4. The automated method of any one of claims 1 to 3, wherein the solution in steps a) and b) has a pH less than 6.5.

5. The automated method of any one of claims 1 to 4, wherein nucleic acids in the eluate collected in steps c)(iii) and d)(iii) comprise at least 50% double-stranded nucleic acids of the total nucleic acids in the eluate.

6. The automated method of any one of claims 1 to 5, further comprising between steps a) and b):
applying a magnetic field to the vessel and removing liquid not bound to the MGPs, thereby leaving double-stranded nucleic acids non-covalently bound to the MGPs; and
adding liquid to the double-stranded nucleic acids non-covalently bound to the MGPs.

7. The automated method of any one of claims 1 to 6, wherein the elution buffer is contacted with the double-stranded nucleic acids non-covalently bound to MGPs for 5-25 minutes in steps c)(i) and d)(i).

8. The automated method of any one of claims 1 to 7, wherein the elution buffer is mixed with the double-stranded nucleic acids non-covalently bound to MGPs using an automated pipetter.

9. The automated method of any one of claims 1 to 8, wherein the double-stranded nucleic acids are from a cell free sample selected from the group consisting of blood, plasma, serum, amniotic fluid, cerebrospinal fluid, and urine.

## Patentansprüche

1. Automatisiertes Verfahren zum Gewinnen doppelsträngiger Nukleinsäuren aus einer flüssigen Probe, umfassend:
a) Inkontaktbringen der flüssigen Probe, die doppelsträngige Nukleinsäuren enthält, mit magnetischen Glaspartikeln (MGPs) in chaotroper Lösung bei einer Temperatur zwischen 25 °C und 45 °C in einem Gefäß, wodurch ermöglicht wird, dass die doppelsträngigen Nukleinsäuren nicht-kovalent an die MGPs in Lösung in dem Gefäß binden;
b) Anlegen eines Magnetfeldes an das Gefäß, das doppelsträngige Nukleinsäuren hält, die nicht-kovalent an MGPs in Lösung gebunden sind, und Entfernen von Flüssigkeit, die nicht an die MGPs gebunden ist;
c) Durchführen eines ersten Elutionsschrittes, wobei der erste Elutionsschritt Folgendes umfasst
(i) Inkontaktbringen doppelsträngiger Nukleinsäuren, die nicht-kovalent an MGPs gebunden sind, mit Elutionspuffer mit einem pH von 7 - 10 in dem Gefäß bei einer Temperatur zwischen 25 °C und 45 °C für 5 - 30 Minuten;
(ii) Anlegen eines Magnetfeldes an das Gefäß; und
(iii) Sammeln von Flüssigkeit, die nicht an die MGPs gebunden ist, wodurch Eluat gesammelt wird;
d) Durchführen eines zweiten Elutionsschrittes, wobei der zweite Elutionsschritt Folgendes umfasst
(i) Inkontaktbringen doppelsträngiger Nukleinsäuren, die nicht-kovalent an MGPs gebunden sind, mit Elutionspuffer mit einem pH von 7 - 10 in dem Gefäß bei einer Temperatur zwischen 25 °C und 45 °C für 5 - 30 Minuten;
(ii) Anlegen eines Magnetfeldes an das Gefäß; und
(iii) Sammeln von Flüssigkeit, die nicht an die MGPs gebunden ist, wodurch Eluat gesammelt wird;
wobei das in den Schritten c)(iii) und d)(iii) gesammelte Eluat mindestens 30 % doppelsträngige Nukleinsäuren aller Nukleinsäuren umfasst, die in dem Eluat aus den MGPs gewonnen wurden, und wobei die doppelsträngigen Nukleinsäuren zellfreie Nukleinsäure mit einer Länge von 50 - 500 Basenpaaren sind.

2. Automatisiertes Verfahren nach Anspruch 1, wobei die Temperatur in den Schritten c)(i) und d)(i) zwischen 30 °C und 45 °C liegt.

3. Automatisiertes Verfahren nach einem der Ansprüche 1 bis 2, wobei die Lösung in den Schritten a) und b) einen pH von weniger als 7 hat.

4. Automatisiertes Verfahren nach einem der Ansprüche 1 bis 3, wobei die Lösung in den Schritten a) und b) einen pH von weniger als 6,5 hat.

5. Automatisiertes Verfahren nach einem der Ansprüche 1 bis 4, wobei Nukleinsäuren in dem in den Schritten c)(iii) und d)(iii) gesammelten Eluat mindestens 50 % doppelsträngige Nukleinsäuren aller Nukleinsäuren in dem Eluat umfassen.

6. Automatisiertes Verfahren nach einem der Ansprüche 1 bis 5, ferner umfassend zwischen den Schritten a) und b):
Anlegen eines Magnetfeldes an das Gefäß und Entfernen von Flüssigkeit, die nicht an die MGPs gebunden ist, wodurch doppelsträngige Nukleinsäuren zurückbleiben, die nicht-kovalent an die MGPs gebunden sind;
und
Zugeben von Flüssigkeit zu den doppelsträngigen Nukleinsäuren, die nicht-kovalent an die MGPs gebunden sind.

7. Automatisiertes Verfahren nach einem der Ansprüche 1 bis 6, wobei der Elutionspuffer in den Schritten c)(i) und d)(i) für 5 - 25 Minuten mit den doppelsträngigen Nukleinsäuren, die nicht-kovalent an MGPs gebunden sind, in Kontakt gebracht wird.

8. Automatisiertes Verfahren nach einem der Ansprüche 1 bis 7, wobei der Elutionspuffer unter Verwendung eines automatisierten Pipettierers mit den doppelsträngigen Nukleinsäuren, die nicht-kovalent an MGPs gebunden sind, gemischt wird.

9. Automatisiertes Verfahren nach einem der Ansprüche 1 bis 8, wobei die doppelsträngigen Nukleinsäuren aus einer zellfreien Probe stammen, die aus der Gruppe ausgewählt ist, die aus Blut, Plasma, Serum, Fruchtwasser, Zerebrospinalflüssigkeit und Urin besteht.

## Revendications

1. Méthode automatisée pour récupérer des acides nucléiques double brin d'un échantillon liquide, comprenant :
a) la mise en contact de l'échantillon liquide contenant des acides nucléiques double brin avec des particules de verre magnétiques (PVM) dans une solution chaotropique à une température comprise entre 25 °C et 45 °C dans un récipient, permettant ainsi aux acides nucléiques double brin de se lier de manière non covalente aux PVM en solution dans le récipient ;
b) l'application d'un champ magnétique au récipient contenant les acides nucléiques double brin liés de manière non covalente aux PVM en solution et l'élimination du liquide non lié aux PVM ;
c) la mise en oeuvre d'une première étape d'élution, dans laquelle ladite première étape d'élution comprend
(i) la mise en contact des acides nucléiques double brin liés de manière non covalente aux PVM avec un tampon d'élution ayant un pH de 7 à 10 dans le récipient à une température comprise entre 25 °C et 45 °C pendant 5 à 30 minutes ;
(ii) l'application d'un champ magnétique au récipient ; et
(iii) la collecte du liquide non lié aux PVM, collectant ainsi l'éluat ;
d) la mise en oeuvre d'une seconde étape d'élution, dans laquelle ladite seconde étape d'élution comprend
(i) la mise en contact des acides nucléiques double brin liés de manière non covalente aux PVM avec un tampon d'élution ayant un pH de 7 à 10 dans le récipient à une température comprise entre 25 °C et 45 °C pendant 5 à 30 minutes ;
(ii) l'application d'un champ magnétique au récipient ; et
(iii) la collecte du liquide non lié aux PVM, collectant ainsi l'éluat ;
dans laquelle l'éluat collecté dans les étapes c)(iii) et d)(iii) comprend au moins 30 % d'acides nucléiques double brin récupérés des PVM de la totalité des acides nucléiques de l'éluat et dans laquelle les acides nucléiques double brin sont un acide nucléique acellulaire de 50 à 500 paires de bases de long.

2. Méthode automatisée selon la revendication 1, dans laquelle la température dans les étapes c)(i) et d)(i) est comprise entre 30 °C et 45 °C.

3. Méthode automatisée selon l'une quelconque des revendications 1 à 2, dans laquelle la solution dans les étapes a) et b) a un pH inférieur à 7.

4. Méthode automatisée selon l'une quelconque des revendications 1 à 3, dans laquelle la solution dans les étapes a) et b) a un pH inférieur à 6,5.

5. Méthode automatisée selon l'une quelconque des revendications 1 à 4, dans laquelle les acides nucléiques dans l'éluat collectés dans les étapes c)(iii) et d)(iii) comprennent au moins 50 % d'acides nucléiques double brin de la totalité des acides nucléiques de l'éluat.

6. Méthode automatisée selon l'une quelconque des revendications 1 à 5, comprenant en outre entre les étapes a) et b) :
l'application d'un champ magnétique au récipient et l'élimination du liquide non lié aux PVM, laissant ainsi les acides nucléiques double brin liés de manière non covalente aux PVM ;
et
l'ajout de liquide aux acides nucléiques double brin liés de manière non covalente aux PVM.

7. Méthode automatisée selon l'une quelconque des revendications 1 à 6, dans laquelle le tampon d'élution est mis en contact avec les acides nucléiques double brin liés de manière non covalente aux PVM pendant 5 à 25 minutes dans les étapes c)(i) et d)(i).

8. Méthode automatisée selon l'une quelconque des revendications 1 à 7, dans laquelle le tampon d'élution est mélangé avec les acides nucléiques double brin liés de manière non covalente aux PVM en utilisant un pipetteur automatisé.

9. Méthode automatisée selon l'une quelconque des revendications 1 à 8, dans laquelle les acides nucléiques double brin proviennent d'un échantillon exempt de cellules choisi dans le groupe constitué par le sang, le plasma, le sérum, le liquide amniotique, le liquide cérébrospinal et l'urine.
